# EUROPÄISCHE PATENTANMELDUNG

(11) **EP 2 382 932 A1**
(43) Veröffentlichungstag der Anmeldung: **02.11.2011**
(21) Anmeldenummer: 11003506.0
(22) Anmeldetag: 29.04.2011
(51) Int. Cl.: A61B 17/34, A61B 19/00

(54) **Trokar**

(30) Priorität: 29.04.2010 DE 102010016704
(71) Anmelder: Trokamed GmbH, 78187 Geisingen (DE)
(72) Erfinder: Tröndle, Karlheinz, 78187 Geisingen (DE); Hartmann, Jürgen, 78194 Immendingen-Hattingen (DE)
(74) Vertreter: Arat, Dogan

(57) **Zusammenfassung**

Die Erfindung betrifft einen Trokar mit einer beweglichen Klappe zum verschliessen einer Trokarhülse. Die bewegliche Klappe kann automatisch mit Hilfe einer Arretiereinrichtung offen gehalten werden.

## Beschreibung

### Technisches Gebiet

Die Erfindung betrifft einen Trokar nach dem Oberbegriff des Anspruchs 1 und zwei Verfahren nach den nebengeordneten Ansprüchen.

### Stand der Technik

Trokare dienen dazu, Operationsinstrumente für minimalinvasive chirurgische Eingriffe beispielsweise in die Bauchhöhe von Patienten einzuführen. Dazu wird eine Trokarhülse an die Bauchdecke angesetzt, ein Trokardorn (Obtruator) in die hohle Trokarhülse eingeschoben, mit Hilfe des Trokardorns eine Öffnung in die Bauchdecke gestossen und anschliessend die Trokarhülse durch die Öffnung in den Bauchraum eingeführt. Anschliessend kann der Trokardorn wieder aus der Trokarhülse herausgezogen werden, und andere chirurgische Instrumente können durch die in der Bauchdecke steckende Trokarhülse in den Bauchraum des Patienten eingeführt werden.

Die meisten Trokarhülsen sind in ihrer Längsrichtung durch einen Ventilkörper verschliessbar, der durch ein in die Trokarhülse eingeschobenes medizinisches Instrument geöffnet werden kann und sich beim Herausziehen des Instruments selbsttätig wieder schliesst. In der Praxis haben sich als Ventilkörper sogenannte automatische Klappenventile bewährt.

Ein gattungsgemässes Klappenventil wird in der DE 10 2005 042 892 A1 beschrieben. Der hier offenbarte, als Klappe ausgebildete Ventilkörper zum Öffnen und Schliessen eines Instruments oder Strömungskanals ist verschwenkbar auf einer im Gehäuse gelagerten Schwenkachse angeordnet. Durch ein Federelement wird die Klappe in Richtung ihrer Schliessstellung vorgespannt, um ein versehentliches Öffnen des Ventilkörpers zu verhindern.

Wie in der DE 43 062 05 C1 beschrieben, können solche Klappen zum Verschliessen von Trokarhülsen aus Metall, Kunststoff oder einer Kombination von beiden aufgebaut sein.

Die in der DE 43 062 05 C1 beschriebenen Klappen können lediglich durch in die Trokarhülse eingeführte medizinische Instrumente geöffnet werden. Ein Klappenventil, welches zusätzlich manuell durch ein Drehelement geöffnet werden kann, wird in der DE 197 12 726 C1 beschrieben. Die Möglichkeit eines manuellen Öffnens der Ventilklappe hat den Vorteil, dass empfindliche medizinische Instrumente nicht durch das Aufdrücken der Klappe beschädigt werden.

Das Dokument WO 03/011153 A1 offenbart einen Trokarkopf mit Klappenventil, welches durch einen externen Hebel geöffnet werden kann. Ventileinrichtungen zum Verschliessen von Trokarhülsen müssen nicht als einteilige Klappe aufgebaut sein, sondern können auch, wie beispielsweise in der EP 1 520 539 B1 beschrieben, aus mehreren Klappenelementen aufgebaut sein. In diesem Dokument wird ein Trokar mit einer Verschliesseinrichtung aus mehreren halbkreisförmigen Klappen beschrieben.

Bei all diesen Klappensystemen zum Verschliessen von Trokarhülsen werden die Klappen beim Herausziehen des eingeführten medizinischen Instruments oder beim Loslassen der Betätigungseinrichtung zum Öffnen der Klappe automatisch wieder verschlossen. Dieses automatische Verschliessen der Klappe kann bei bestimmten medizinischen Eingriffen von Nachteil sein. Beispielsweise bei der Verwendung komplexer Instrumente zur minimalinvasiven Chirurgie, bei Entnahme von Gewebe mit grossem Volumen oder bei Morcellations-Anwendungen wären Klappen von Vorteil, die sich nicht automatisch wieder verschliessen, sondern automatisch offen gehalten werden können. Die Möglichkeit zur Arretierung einer Trokarhülsenklappe im Offenzustand wird in der DE 296 07 146 U1 erwähnt. Ein technisches Beispiel für eine solche Arretiereinrichtung wird jedoch nicht gegeben.

### Offenbarung der Erfindung

Es ist Aufgabe der Erfindung, die Nachteile des oben genannten Standes der Technik zu beheben oder zumindest zu vermindern, insbesondere ist es Aufgabe der Erfindung, eine Arretiereinrichtung für Klappen in Trokarhülsen zu verbessern.

Die Aufgabe wird durch einen Trokar gemäss Anspruch 1 und die Verfahren gemäss den nebengeordneten Ansprüchen gelöst.

Die Erfindung hat gegenüber dem Stand der Technik den Vorteil, dass eine Klappe zum Verschliessen einer Trokarhülse nicht nur in Schliessrichtung vorgespannt und somit automatisch verschlossen werden, sondern auch in einer Arretierposition arretiert und somit automatisch offen gehalten werden kann. Dadurch werden bestimmte medizinische Eingriffe wie zum Beispiel Morcellations-Anwendungen oder Gewebeentnahmen enorm erleichtert.

Erfindungsgemäss ist ein Trokar mit einer Trokarhülse vorgesehen, in welcher eine bewegliche Klappe zum Verschliessen der Trokarhülse in Längsrichtung angeordnet ist. Die Trokarhülse ist in bevorzugten Ausführungsformen mehrteilig aufgebaut und umfasst ein Schaftrohr, ein Gehäuse, einen Hauptteil einen Hahnansatz, eine Dichtkappenaufnahme und eine Dichtkappe. An der Klappe ist eine Arretiereinrichtung angeordnet, welche bei Erreichen eines Mindestöffnungswinkels der Klappe, gemessen zwischen der Klappe und der Querachse der Trokarhülse, die Klappe automatisch in eine Arretierposition bringt und somit offen hält. Bei der bevorzugen Ausführungsform ist die Klappe in der Arretierposition leicht entlang der Querachse der Trokarhülse gegenüber einer Schliessposition verschoben. Bei typischen Ausführungsformen erfolgt das Arretieren in der Arretierposition nicht automatisch sondern durch manuelles Verschieben. Vorteilhafte Ausführungsformen zeichnen sich dadurch aus, dass die Klappe nicht automatisch in eine Arretiereinrichtung geschoben, sondern automatisch in einer Offenposition gehalten wird.

Beim bevorzugten Ausführungsbeispiel ist der Mindestöffnungswinkel grösser gleich 60° und kleiner gleich 20°, bevorzugt werden Mindestöffnungswinkel grösser gleich 70° und kleiner gleich 110°, besonders bevorzugt sind Mindestöffnungswinkel grösser gleich 80° und kleiner gleich 100°, wobei Mindestöffnungswinkel grösser gleich 85° und kleiner gleich 95° besonders bevorzugt sind.

Im bevorzugten Ausführungsbeispiel ist zwischen der Trokarhülse und der Klappe ein Kraftspeicher, bevorzugt Feder, beispielsweise eine Druckfeder oder eine Zugfeder, angeordnet, welcher die Klappe bei Erreichen des Mindestöffnungswinkels automatisch in die Arretierposition drückt. Unter Kraftspeicher oder Feder wird hier ein Bauteil verstanden, welches unter Belastung nachgibt und nach Entlastung in die ursprüngliche Gestalt zurückkehrt, sich also elastisch verhält.

Bei vorteilhaften Ausführungsformen ist zwischen der Trokarhülse und der Klappe eine Schliessfeder angeordnet, welche die Klappe in Schliessrichtung vorspannt. Diese Schliessfeder bringt die Klappe automatisch in ihre Schliessposition, sofern die Klappe nicht gerade in der Arretierposition oder der Offenposition gehalten wird.

Bei der bevorzugten Ausführungsform ist an der Trokarhülse eine Betätigungseinrichtung zum manuellen Verschwenken der Klappe angeordnet. Vorzugsweise ist zwischen Klappe und Betätigungseinrichtung eine Drehachse angeordnet. Bei bevorzugten Ausführungsformen umfasst die Betätigungseinrichtung einen Hebel. Vorzugsweise kann durch Drücken der Betätigungseinrichtung die Klappe stufenlos in Richtung der Offenposition verschwenkt werden. Dieses Verschwenken der Klappe ist jedoch vorzugsweise nicht nur mit Hilfe der Betätigungseinrichtung, sondern auch durch ein in die Trokarhülse eingeführtes Instrument möglich.

Bei bevorzugten Ausführungsformen umfasst die Betätigungseinrichtung, bevorzugt der Hebel, eine Einkerbung in Form einer Fingerkuppe. Dies hat den Vorteil, dass ein Abrutschen des Fingers bei Betätigung der Betätigungseinrichtung verhindert wird.

Bei der bevorzugten Ausführungsform umfasst die Arretiereinrichtung einen Arretierschlitz, in welchen die Klappe bei Erreichen des Mindestöffnungswinkels zumindest teilweise eingeschoben wird. Vorzugsweise verläuft dieser Arretierschlitz im Wesentlichen parallel zur Längsachse der Trokarhülse. Ein solcher Arretierschlitz hat den Vorteil, dass die Klappe sehr einfach in der Arretierposition gehalten werden kann. Bei vorteilhaften Ausführungsformen verläuft der Arretierschlitz nicht im Wesentlichen parallel zur Längsachse der Trokarhülse, sondern hat eine andere Ausrichtung bezüglich zur Längsrichtung der Trokarhülse.

Bei typischen Ausführungsformen wird die Klappe bei Erreichen des Mindestöffnungswinkeis nicht automatisch in einen Arretierschlitz geschoben, sondern automatisch in einer Offenposition gehalten. Bei vorteilhaften Ausführungsformen wird das Halten in der Offenposition durch eine Blockierbuchse erreicht, welche sich bei Erreichen des Mindestöffnungswinkels zumindest teilweise über oder in die Klappe schiebt.

Bei besonders vorteilhaften Ausführungsformen wird die Klappe gleichzeitig in einen Arretierschlitz verbracht und von einer Blockierbuchse festgehalten.

Bei vorteilhaften Ausführungsformen ist an der Trokarhülse eine Signaleinrichtung zum Anzeigen der Arretierung angeordnet. Bei besonders vorteilhaften Ausführungsformen umfasst die Signaleinrichtung einen Farbring. Dies hat den Vorteil, dass vom Operateur einfach erkannt werden kann, ob die Klappe arretiert oder freigegeben ist.

Beim typischen Betrieb des Trokars wird die Klappe entweder durch manuelles Betätigen der Betätigungseinrichtung oder durch Einführen eines medizinischen Instruments stufenlos geöffnet. Sobald die Klappe den Mindestöffnungswinkel erreicht, wird sie automatisch zumindest teilweise in den Arretierschlitz verschoben. Dort wird die Klappe dann in der Arretierposition gehalten. Vorzugsweise wird die Klappe dann durch Betätigen der Betätigungseinrichtung aus der Arretierposition gelöst, wenn eine geöffnete Klappe nicht weiter notwendig ist. Das Lösen der Klappe geschieht vorzugsweise dadurch, dass die Betätigungseinrichtung im Wesentlichen in Querrichtung der Trokarhülse gedrückt wird. Bei vorteilhaften Ausführungsformen führt eine andere Art der Betätigung des Betätigungselements, z. B. Ziehen, Drehen oder Anheben, zum Lösen der Klappe aus der Arretierposition.

Bei einem vorteilhaften Verfahren zum Arretieren der Klappe wird die Klappe zuerst durch manuelles Betätigen der Betätigungseinrichtung oder durch ein in die Trockarhülse eingeführtes Instrument sukzessive aus der Schliessposition heraus geöffnet. Sobald der Mindestöffnungswinkel erreicht wird, schiebt sich eine Blockierbuchse zumindest teilweise über oder in die Klappe. Dadurch kann sich die Klappe nun nicht mehr schliessen und wird in einer Offenposition gehalten. Typischerweise erfolgt das Lösen der Klappe aus der Offenposition durch Betätigen der Betätigungseinrichtung, vorzugsweise durch Drücken der Betätigungseinrichtung im Wesentlichen in Querrichtung der Trokarhülse. Bei vorteilhaften Ausführungsformen führt eine andere Art der Betätigung des Betätigungselements, z. B. Ziehen, Drehen oder Anheben, zum Lösen der Klappe aus der Arretierposition.

### Kurze Beschreibung der Zeichnungen

Nachfolgend wird die Erfindung anhand schematischer Zeichnungen näher erläutert, die zeigen:
Figur 1: eine Aussenansicht einer Trokarhülse mit einem Hebel,
Figur 2: einen Querschnitt durch die Trokarhülse nach Fig.1 entlang der Linie II-II auf Höhe des Hebels und einer Arretiereinrichtung,
Figur 3: einen Querschnitt durch die Trokarhülse auf Höhe des Hebels und der Arretiereinrichtung bei geöffneter Klappe und
Figur 4: einen Querschnitt durch die Trokarhülse auf Höhe des Hebels und der Arretiereinrichtung bei geöffneter Klappe in einer Ausführungsform mit Blockierbuchse,
Figur 5: eine schematische Seitenansicht eines Teilbereichs der erfindungsgemässen Trokarhülse in eingerasteter Offenposition der Klappe,
Figur 6: eine schematische Seitenansicht wie in Figur 5 mit geschlossener Klappe.

### Beschreibung eines bevorzugten Ausführungsbeispiels

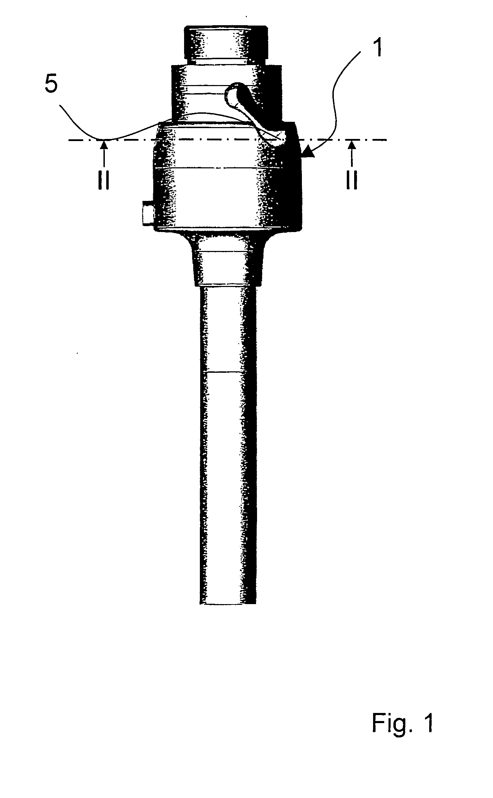

Figur 1 zeigt eine Trokarhülse 1 eines nicht näher beschriebenen Trokars mit einer als Hebel ausgebildeten Betätigungseinrichtung 5, welche eine fingerkuppenförmige Einkerbung aufweist.

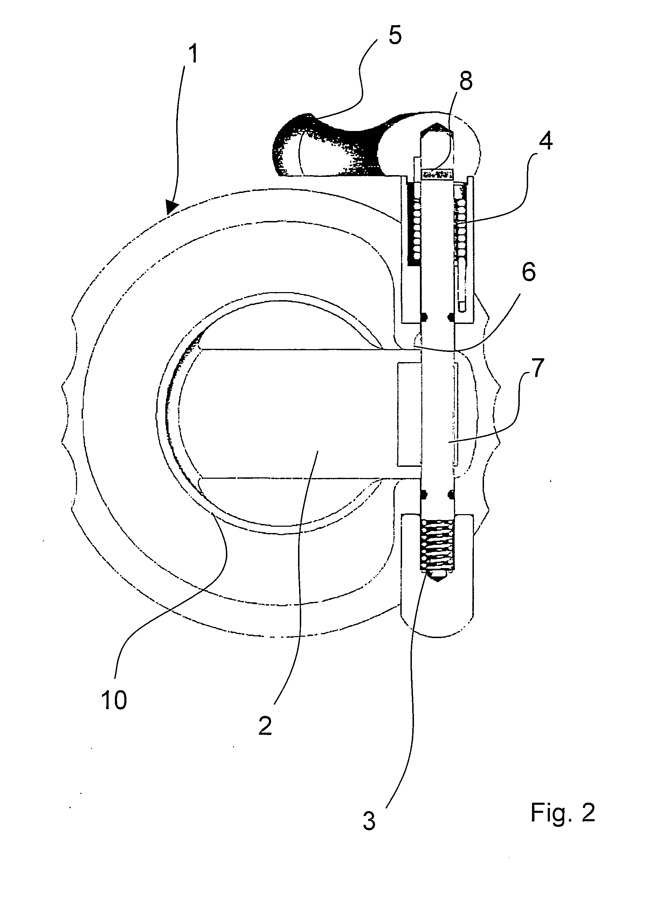

Figur 2 zeigt einen Querschnitt durch die Trokarhülse 1, bei dem eine Klappe 2 zum Verschliessen der Trokarhülse 1 erkennbar ist. Die Klappe 2 ist in ihrer Schliessposition dargestellt. In dieser Schliessposition verschliesst sie einen Durchlass durch die Trokarhülse, indem sie einem Dichtring 10 aufliegt. Über eine Drehachse 7 ist die Klappe 2 mit der Betätigungseinrichtung 5 verbunden. Eine Schliessfeder 4 spannt Drehachse 7 und Klappe 2 gegen die Trokarhülse in Schliessrichtung der Klappe 2 vor. Durch Betätigen der Betätigungseinrichtung 5 kann die Klappe 2 sukzessive stufenlos geöffnet werden.

Wenn die Klappe einen Mindestöffnungswinkel erreicht, wird sie von einer Druckfeder 3 automatisch in einen Arretierschlitz 6 gedrückt. Dort wird die Klappe 2 dann so lange automatisch arretiert, bis der Arretiermechanismus über die Betätigungseinrichtung 5 gelöst wird.

Auf der Drehachse 7 ist eine Signaleinrichtung 8 angeordnet, welche als Farbring ausgebildet ist und welche bei geöffneter Klappe 2 an der Aussenseite der Trokarhülse 1 sichtbar ist. Damit wird dem Benutzer angezeigt, ob die Klappe 2 arretiert ist oder nicht.

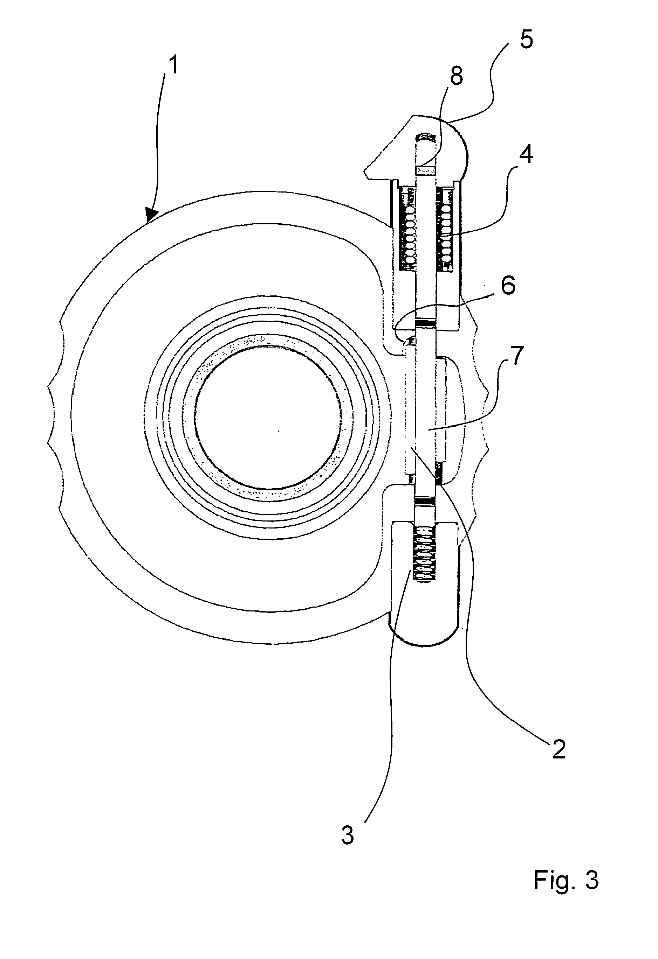

Die Figur 3 zeigt die gleiche Ansicht wie die Figur 2, jedoch ist die Klappe 2 in Figur 3 geöffnet. In Figur 3 ist erkennbar, dass die Klappe 2 teilweise in den Arretierschlitz 6 eingefahren ist und dort automatisch gehalten wird. Erst durch ein Drücken der Betätigungseinrichtung 5 in Querrichtung der Trokarhülse 1 kann die Klappe 2 wieder aus dem Arretierschlitz herausgedrückt werden. Hierbei muss der von der Druckfeder 3 aufgebrachten Kraft entgegengewirkt werden.

Figur 4 zeigt ein weiteres Ausführungsbeispiel, bei dem die Klappe 2 von einer Blockierbuchse 9, welche teilweise über die Klappe 2 geschoben ist, in einer Offenposition gehalten wird.

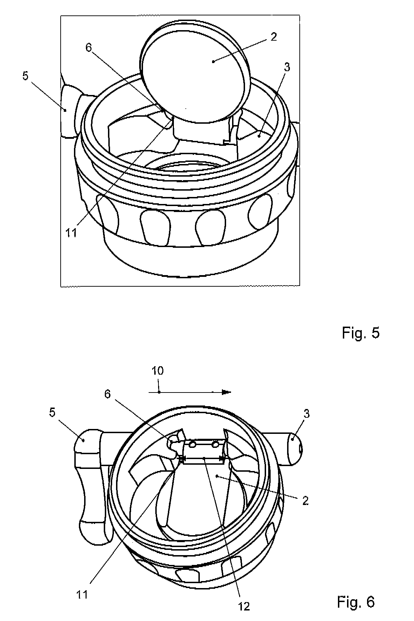

In Figur 5 ist die Klappe 2 in geöffneter Position gezeigt. In dieser geöffneten Position ist gut zu erkennen, wie die Klappe 2 in den Arretierschlitz 6 eingeschoben ist. Dieses Einschieben erfolgt zum Einen durch Betätigung des Hebels 5 und zum Anderen durch die Druckfeder 3. Die Druckfeder 3 ist in Figur 3 zwar nicht gezeigt, aber durch eine Bezugsziffernangabe angedeutet.

In Figur 6 ist eine geschlossene Klappe 2 gezeigt. Von der geöffneten Position, welche in Figur 5 gezeigt wurde, hin zur geschlossenen Position, wie sie in Figur 6 gezeigt ist, kommt der Nutzer dadurch, dass der Hebel 5 gegen die Kraft der Druckfeder 3 in Richtung eines Pfeils 10 gedrückt und zusätzlich gedreht wird. Dadurch löst sich ein Sockelbereich 11 des Deckels 2 aus dem Arretierschlitz 6. Der Sockelbereich 11 ist derart gestaltet, dass er in einer Ausnehmung 12 der Trokarhülse 1 umlegbar oder schwenkbar ist. Beim nochmaligen Öffnen der Klappe 2 wird der Sockelbereich 11 durch Drehen des Hebels um ca. 70° bis 90° geschwenkt. Nach dem Passieren des Sockelbereichs 11 durch die Ausnehmung 12 drückt die Druckfeder 3 den Sockelbereich 11 oder vielmehr den Deckel 2 in den Arretierschlitz 6.

Die Erfindung ist nicht auf die vorstehend beschriebenen Ausführungsbeispiele beschränkt. Vielmehr wird der Schutzumfang durch die Ansprüche bestimmt.

## Patentansprüche

1. Trokar, mit
- einer Trokarhülse (1), welche mindestens ein Bauelement umfasst,
- einer in der Trokarhülse (1) angeordneten beweglichen Klappe (2) zum Verschliessen der Trokarhülse in Längsrichtung der Trokarhülse (1)
- einer der Klappe (2) zugeordneten Arretiereinrichtung,
**dadurch gekennzeichnet,**
**dass** die Klappe (2) bei Erreichen eines Mindestöffnungswinkels, gemessen zwischen der Klappe (2) und der Querachse der Trokarhülse (1), von der Arretiereinrichtung automatisch in einer Offenposition haltbar ist oder in eine Arretierposition bringbar ist.

2. Trokar nach Anspruch 1, **dadurch gekennzeichnet, dass** der Mindestöffnungswinkel grösser gleich 60 Grad und kleiner gleich 120 Grad ist.

3. Trokar nach einem der vorangehenden Ansprüche, **dadurch gekennzeichnet, dass** eine Druckfeder (3), welche die Klappe (2) gegen die Trokarhülse (1) belastet, derart angeordnet ist, so dass die Klappe (2) bei Erreichen des Mindestöffnungswinkels automatisch in die Arretierposition gedrückt wird.

4. Trokar nach Anspruch 1, **dadurch gekennzeichnet, dass** zwischen Trokarhülse (1) und Klappe (2) eine Schliessfeder (4) derart angeordnet ist, so dass die Klappe in Schliessrichtung vorgespannt wird.

5. Trokar nach Anspruch 1, **dadurch gekennzeichnet, dass** an der Trokarhülse (1) eine Betätigungseinrichtung (5) zum Verschwenken der Klappe (2) angeordnet ist, wobei zwischen der Klappe (2) und der Betätigungseinrichtung (5) eine Drehachse (7) angeordnet ist.

6. Trokar nach einem der vorangehenden Ansprüche, **dadurch gekennzeichnet, dass** die Arretiereinrichtung einen Arretierschlitz (6) umfasst.

7. Trokar nach Anspruch 1, **dadurch gekennzeichnet, dass** die Arretiereinrichtung eine Blockierbuchse (9) umfasst, die derart angeordnet ist, sodass sie bei Erreichen des Mindestöffnungswinkels zumindest teilweise über die Klappe (2) schiebbar ist.

8. Trokar nach einem der vorangehenden Ansprüche, **dadurch gekennzeichnet, dass** an der Trokarhülse (1) eine Signaleinrichtung (8) zum Anzeigen der Arretierung angeordnet ist.

9. Verfahren zum Arretieren und Lösen einer Klappe (2) in einer Trokarhülse (1) nach Anspruch 6, mit den Schritten:
- Manuelles Betätigen der Betätigungseinrichtung (5) zum Öffnen der Klappe (2) oder Aufstossen der Klappe (2) mit einem in die Trokarhülse (1) eingeführten Instrument,
- Zumindest teilweises Verbringen der Klappe (2) in den Arretierschlitz (6), sobald der Mindestöffnungswinkel erreicht wird,
- Automatisches Halten der Klappe (2) in der Arretierposition
- Lösen der Klappe (2) aus der Arretierposition durch Betätigen der Betätigungseinrichtung (5).

10. Verfahren zum Arretieren und Lösen einer Klappe (2) in einer Trokarhülse (1) nach Anspruch 7, mit den Schritten:
- Manuelles Betätigen der Betätigungseinrichtung (5) zum Öffnen der Klappe (2) oder Aufstossen der Klappe (2) mit einem in die Trokarhülse (1) eingeführten Instrument,
- Zumindest teilweises Schieben der Blockierbuchse (9) über die Klappe (2), sobald der Mindestöffnungswinkel erreicht wird,
- Automatisches Halten der Klappe (2) in der Offenposition
- Lösen der Klappe (2) aus der Offenposition durch Betätigen der Betätigungseinrichtung (5).
